(19) Europäisches Patentamt
European Patent Office
Office européen des brevets

(11) Veröffentlichungsnummer : **0 247 507 B1**

(12) # EUROPÄISCHE PATENTSCHRIFT

(45) Veröffentlichungstag der Patentschrift : **09.01.91 Patentblatt 91/02**

(51) Int. Cl.[5] : **A61K 47/00**, A61K 31/635, A61L 15/16

(21) Anmeldenummer : **87107310.2**

(22) Anmeldetag : **20.05.87**

(54) **Zusammensetzung für transdermale therapeutische Systeme von Schleifendiuretika und Verfahren zu ihrer Herstellung.**

(30) Priorität : **27.05.86 DE 3617824**

(43) Veröffentlichungstag der Anmeldung : **02.12.87 Patentblatt 87/49**

(45) Bekanntmachung des Hinweises auf die Patenterteilung : **09.01.91 Patentblatt 91/02**

(84) Benannte Vertragsstaaten : **AT BE CH DE ES FR GB GR IT LI LU NL SE**

(56) Entgegenhaltungen :
**EP-A- 0 038 512**
**EP-A- 0 043 548**
**EP-A- 0 139 127**
**DE-A- 1 492 188**
**DE-A- 3 536 669**
**US-A- 3 175 949**

(73) Patentinhaber : **HOECHST AKTIENGESELLSCHAFT**
**Postfach 80 03 20**
**D-6230 Frankfurt am Main 80 (DE)**

(72) Erfinder : **Hropot, Max, Dr.**
**Friedrich-Stolz-Strasse 13**
**D-6093 Flörsheim am Main (DE)**
Erfinder : **Petri, Walter, Dr.**
**Panoramastrasse 19**
**D-6272 Niedernhausen/Taunus (DE)**
Erfinder : **Reul, Bernhard**
**Blumenstrasse 25**
**D-6240 Königstein/Taunus (DE)**

**EP 0 247 507 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Zur Verbesserung der Patienten-Compliance bei der Behandlung der Hypertonie mit Schleifendiuretika ist es sinnvoll, die initiale Diurese abzuflachen und die Wirkdauer der Wirkstoffe zu verlängern, d.h. das Wirkstoffabgabeprofil aus einer betrachteten Arzneiform heraus sollte konstant und langandauernd sein.

Retard-Zubereitungen zur oralen Applikation, aus welchen der Wirkstoff über einen längeren Zeitraum - etwa bis zu 6 Stunden – freigesetzt wird, wurden z.B. für die Schleifendiuretika Furosemid und Piretanid bereits beschrieben (Deutsche Offenlegungsschriften 23 43 218 [entspr. US-PS 4.324.779] und 26 55 331). Es ist auch schon beschrieben worden, daß in transdermale therapeutische Systeme (TTS) inkorporierte Wirkstoffe zur perkutanen Resorption kontinuierlich und retardiert abgegeben werden. So werden in der Literatur Systeme unterschiedlicher Art mit den Wirkstoffen Nitroglycerin, Scopolamin, Östradiol, Clonidin beschrieben ; sie sind in vielen Ländern seit einiger Zeit auf dem Arzneimittelmarkt.

Versuche mit den bekannten Grundlagen dieser Systeme zeigten, daß die in diesen TTS-Systemen enthaltenen Grundlagen zur Penetration der Schleifendiuretika Furosemid (4-ChlorN-furfuryl-5-sulfamoylanthranilsäure ; Deutsche Patentschrift 11 22 541 entsprechend US-PS 3.058.882), Piretanid [4-Phenoxy-3-(1-pyrrolidinyl)-5-sulfamoylbenzoesäure ; Deutsche Offenlegungsschrift 24 19 970 ; US-PS 4.010.273) und Lemidosul (proposed INN ; 2-Aminomethyl-4(1.1-dimethylethyl)-6-methyl-sulfonylphenol-Hydrochlorid ; Deutsche Offenlegungsschrift 32 08 190 entspr. US-Patentanmeldungs Nr. 472.226) durch die lebende Haut nicht geeignet sind. Die Grundlagen dieser Systeme sind je nach Wirkstoff entweder hydrophile oder lipophile Stoffe.

Es wurde nun überraschend gefunden, daß die genannten Schleifendiuretika und deren physiologisch vertretbare Salze zu TTS mit konstanter Wirkstoffabgabe führen, wenn eine Grundlage aus einem Fettsäurealkanolamid oder einem Fettsäurealkanolamidgemisch und ggf. einem Carbonsäureester oder einem Carbonsäureestergemisch verwendet wird, also eine Grundlage mit stark ausgeprägten lipophilen Eigenschaften neben hydrophilen. Darüber hinaus kommt es insbesondere bei Verwendung von Grundlagen, in denen der Fettsäurealkanolamidanteil größer ist als der Esteranteil zu einer im Vergleich zu oralen Retardzubereitungen deutlich länger andauernden Wirkstoffabgabe.

Die Erfindung betrifft daher eine Zusammensetzung für ein transdermales therapeutisches System, welche dadurch gekennzeichnet ist, daß sie als Wirkstoff Furosemid, Piretanid oder Lemidosul oder ein physiologisch verträgliches Salz dieser Schleifendiuretika in einer Grundlage bestehend aus einem Fettsäurealkanolamid oder einem Fettsäurealkanolamidgemisch mit jeweils 8-18 Kohlenstoffatomen im Fettsäurerest und 2 bis 6 Kohlenstoffatomen und 1 bis 3 Hydroxygruppen im Alkanolamidrest und gegebenenfalls einem Carbonsäureester oder einem Carbonsäureestergemisch mit jeweils 1-22 Kohlenstoffatomen im Carbonsäureteil und 1-22 Kohlenstoffatomen im Alkoholteil enthält.

Als physiologisch verträgliche Salze kommen sowohl wasserlösliche als auch schwerwasserlösliche in Betracht.

In dem vorstehenden und folgenden Ausführungen ist unter einem Fettsäurealkanolamid sowohl ein Fettsäuremonoalkanolamid als auch ein Fettsäuredialkanolamid zu verstehen. Der Fettsäurerest ist gesättigt oder ungesättigt. Die Fettsäurealkanolamide können auch wie folgt bezeichnet werden :

a) N-Alkanoyl-N-alkylamino-hydroxylalkane bzw. N-Alkenoyl-N-alkylamino-hydroxyalkane

b) N-Alkanoyl-N-hydroxyalkylamino-hydroxyalkane bzw. N-Alkenoyl-N-hydroxyalkylamino-hydroxyalkane und

c) N-Alkanoyl-amino-hydroxyalkane bzw. N-Alkenoyl-amino-hydroxyalkane.

Bei den Hydroxygruppen handelt es sich um primäre, sekundäre oder tertiäre Hydroxygruppen. Die erfindungsgemäßen Zusammensetzungen enthalten z.B. Fettsäurealkanolamide der folgenden Formeln, wobei R den Rest der Fettsäure darstellt :

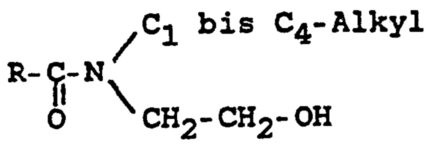

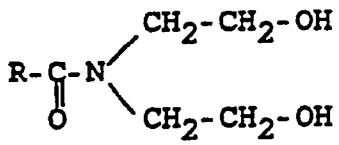

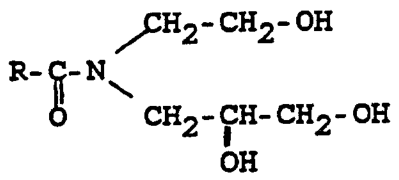

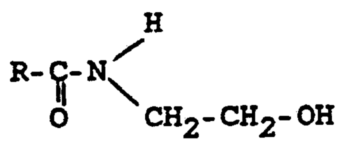

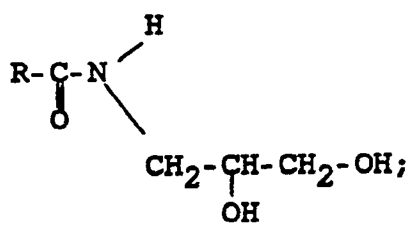

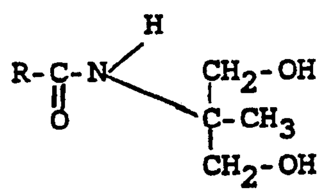

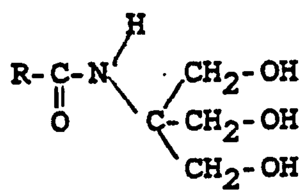

Als Carbonsäureester kommen Ester mit 1-, 2- und 3-wertigen Alkoholen in Betracht. Bei den Alkoholen handelt es sich um primäre, sekundäre sowie tertiäre Alkohole. Die Carbonsäuren bzw. Alkohole sind gesättigt oder ungesättigt.

Vorzugsweise werden Ester aus aliphatischen Monocarbonsäuren und aliphatischen Alkoholen verwendet.

Die Grundlage besteht vorzugsweise aus einem Fettsäurealkanolamid, wie z. B. Fettsäurediethanolamid oder Fettsäuremonoisobutandiolamid, und einem Carbonsäureester ; die Säurereste der beiden Komponenten sind vorzugsweise gleich. Besonders geeignet sind Caprinsäurediethanolamid, Laurinsäurediethanolamid und Caprinsäuremonoisobutandiolamid und Caprinsäureethylester, Laurinsäurehexylester. Falls Estergemische verwendet werden, eignen sich insbesondere Gemische aus Caprinsäureethylester und Essigsäuredecylester.

Die erfindungsgemäßen Zusammensetzungen können außer dem Wirkstoff und der Grundlage weitere inerte Füllstoffe oder übliche Hilfsstoffe wie z.B Wasser, Paraffine, kolloidale Kieselsäure oder oberflächenaktive Stoffe enthalten.

Je nachdem wie das Mischungsverhältnis von Fettsäurealkanolamid und Carbonsäureester ist, entstehen Penetrationssysteme mit unterschiedlich ausgeprägter retardierter Wirkstoffabgabe. Die Penetration

des Wirkstoffs durch die Haut hängt daher sowohl von diesem Mischungsverhältnis als auch von der Beschaffenheit der Haut sowie von der Größe der Kontaktfläche ab.

Die erfindungsgemäßen Zusammensetzungen enthalten vorzugsweise 1 bis 50 Gew.-% Wirkstoff bezogen auf das Gesamtgewicht der Zusammensetzung. Das Mischungsverhältnis von Fettsäurealkanolamid zu Carbonsäureester in der Grundlage beträgt z.B. 2 zu 98 bis 98 : 2. Je höher der Fettsäurealkanolamidanteil ist, desto größer ist die Retardierung.

Falls die Zusammensetzung noch einen oder mehrere Füllstoffe oder übliche Hilfsstoffe enthält, so beträgt deren Anteil zweckmäßigerweise 1 bis 90 Gew.-% (bezogen auf das Gesamtgewicht der Zusammensetzung).

Die Erfindung betrifft ferner ein Verfahren zur Herstellung der Zusammensetzungen sowie deren Verwendung in transdermalen therapeutischen Systemen. Das Verfahren ist dadurch gekennzeichnet, daß man Furosemid, Piretanid oder Lemisodul oder ein physiologisch verträgliches Salz dieser Schleifendiuretika mit einen Fettsäurealkanolamid oder einem Fettsäurealkanolamidgemisch mit jeweils 8 bis 18 Kohlenstoffatomen im Fettsäurerest und 2 bis 6 Kohlenstoffatomen und 1 bis 3 Hydroxygruppen im Alkanolamidrest und gegebenenfalls einem Carbonsäureester oder einem Carbonsäureestergemisch mit jeweils 1-22 Kohlenstoffatomen im Carbonsäureteil und 1-22 Kohlenstoffatomen im Alkoholteil bei Temperaturen zwischen Raumtemperatur und 50°C innig vermischt.

Bei der Herstellung der Mischung kann man den Wirkstoff in dem Carbonsäureester oder dem Carbonsäureestergemisch suspendieren oder lösen, zur Suspension oder Lösung das Fettsäurealkanolamid geben und anschließend bei Temperaturen zwischen Raumtemperatur und 50°C innig vermischen. Man kann auch die genannten Wirkstoffe zunächst mit dem Fettsäurealkanolamid bei Temperaturen zwischen Raumtemperatur und 50°C mischen und danach den Carbonsäureester oder das Carbonsäureestergemisch zusetzen. Die Mischung kann z.B. in einem üblichen Rührwerk mit eingebautem Homogenisator durchgeführt werden.

Die erfindungsgemäßen Zusammensetzungen sind fest, halbfest (pastös) oder flüssig und eignen sich für die Verwendung in transdermalen therapeutischen Systemen. Solche Systeme sind z.B. Folien oder Pflaster mit runden oder ovalen Kontaktflächen von 0,5 bis 25 $cm^2$ Größe oder vorzugsweise Pflasterpackungen mit Arzneimittelbehälter (Napf). Besonders geeignet ist eine Pflasterpackung, welche acs einer aus 2 Lagen gebildeten Hülle, die die Zusammensetzung einschließt, und einer hauthaftfähigen, elastischen Folie besteht. Solche Pflasterpackungen sind z.B. in der Deutschen Offenlegungsschrift 32 04 582 beschrieben. Eine andere Ausführungsform einer geeigneten Pflasterpackung wird in dem deutschen Gebrauchsmuster 84 07 36 beschrieben. Diese Pflasterpackungen besitzen Arzneimittelbehältner zur Aufnahme von festen, halbfesten oder flüssigen Zusammensetzungen. Falls in eine solche Pflasterpackung eine halbfeste oder flüssige erfindungsgemäße Zusammensetzung eingearbeitet wird, empfiehlt es sich, eine aufsaugende Textileinlage zusätzlich einzusetzen. Aus den erfindungsgemäßen Zusammensetzungen werden die Wirkstoffe retardiert und kontinuierlich percutan abgegeben. Die Bioverfügbarkeit des Wirkstoffs ist z.T. besser als diejenige nach oraler Applikation. Ein weiterer Vorteil besteht in der Vermeidung der Magen-Darmpassage, so daß die neue Applikationsform eine problemlose, für einen großen Patientenkreis geeignete Darreichungsform bedeutet. Aufgrund des Freigabeprofils eignen sich die erfindungsgemäßen Zusammensetzungen nicht nur als Diuretika sondern auch für die Behandlung des Bluthochdrucks. Die Dosierungseinheit, also ein transdermales therapeutisches System, enthält vorzugsweise 5 bis 500 mg Wirkstoff, insbesondere 5 bis 200 mg.

Die folgenden Beispiele sollen die Erfindung erläutern. Die Zusammensetzungen wurden durch Mischen der Wirkstoffe mit der Grundlage in einem Rührwerk mit eingebautem Homogenisator erhalten. Als transdermale therapeutische Systeme wurden aus dem deutschen Gebrauchsmuster 84 07 036 bekannte Pflasterpackungen verwendet.

Die Mengenangaben in den Beispielen beziehen sich jeweils auf die Zusammensetzung für eine Dosiereinheit.

## Beispiel 1

### Zusammensetzung für eine Dosiereinheit

| | | |
|---|---|---|
| a) | Piretanid | 20.0 mg |
| | Laurinsäurediethanolamid [Laurinsäure-N-bis-(2-hydroxy-ethylamid)] | 64.0 mg |
| | Laurinsäurehexylester | 16.0 mg |
| | | 100.0 mg |

| | | |
|---|---|---|
| b) | Piretanid | 20.0 mg |
| | Laurinsäurediethanolamid | 24.0 mg |
| | Laurinsäurehexylester | 56.0 mg |
| | | 100.0 mg |

Hinsichtlich der Natriurese an der behaarten Ratte zeigte die Zusammensetzung a) (Mischungsverhältnis Laurinsäurediethanolamid : Laurinsäurehexylester = 8 : 2) in den ersten 5 Stunden nach percutaner Applikation eine mäßige natriuretische Wirkung, die mit einem langsamen Anstieg der Blutspiegel von Piretanid einherging. Erst in der Sammelperiode 6. - 24. Std. kam es zu einer starken Natriurese, die mit dem maximalen Blutspiegel von Piretanid in der 6. Stunde korrelierte. Das zeitliche Wirkprofil des Schleifendiuretikums Piretanid konnte in o.g. Präparation im Sinne einer verlängerten Wirkung verändert werden (vgl. Tabellen 1 und 2).

Im Gegensatz zu Zusammensetzung a) zeigte die Präparation b) (Mischungsverhältnis Laurinsäurediethanolamid : Laurinsäurherexylrster = 3 : 7) eine starke natriuretische Wirkung in der 1. - 5. Stunden-Sammelperiode, die mit einem starken Anstieg des Blutspiegels von Piretanid einher ging, dessen Maximum bereits in der vierten Stunde nach Applikation erreicht wurde. Danach fiel der Blutspiegel von Piretanid schneller ab als der nach Applikation von a), so daß die natriuretische Wirkung von b) in der 6 - 24 Stunden-Sammelperiode deutlich schwächer war als von a) ; eine verlängerte Wirkung von Piretanid war nicht vorhanden (vgl. Tabellen 1 und 2). Im Falle der Präparation b) entspricht das zeitliche Wirkprofil von Piretanid dem Wirkprofil nach oraler Gabe (Piretanid suspendiert in 2%iger wäßriger Stärkelösung) und somit dem eines typischen Schleifendiuretikums (vgl. Tabelle 3).

Tabelle 1

Vergleich des Blutspiegels (µg/ml) von Piretanid nach dessen percutaner Applikation an der Ratte (n = 6)

| Std. | Zusammensetzung a) 20 mg/Ratte | Zusammensetzung b) 20 mg/Ratte |
|---|---|---|
| 1 | 0 | 19.3 ± 25.0 |
| 2 | 12.7 ± 12.6 | 63.8 ± 57.6 |
| 4 | 40.7 ± 19.1 | 75.5 ± 49.0 |
| 6 | 62.8 ± 32.2 | 62.2 ± 34.0 |
| 8 | 50.8 ± 18.9 | 58.7 ± 25.8 |
| 24 | 33.7 ± 17.9 | 20.6 ± 8.6 |
| 28 | 21.3 ± 13.0 | 0 |
| 32 | 26.8 ± 11.3 | 0 |
| 48 | 54.7 ± 29.9 | 0 |

Tabelle 2

**Salidiuretische Wirkung von TTS-Piretanid an der Ratte**

| Präparat | Sammel-perioden | Urin ml/kg | Natrium mmol/kg | Kalium mmol/kg | Chlorid mmol/kg |
|---|---|---|---|---|---|
| a) Piretanid 20 mg/Ratte p.c. (n = 6) | 1 - 5 Std. | 23.5 ± 10.7 | 3.33 ± 2.16 | 1.29 ± 0.46 | 3.20 ± 1.70 |
| | 6 - 24 Std. | 40.3 ± 6.0 | 6.36 ± 1.48 | 4.68 ± 0.51 | 5.70 ± 1.78 |
| | 1 - 24 Std. | 63.8 ± 8.0 | 9.69 ± 1.86 | 5.97 ± 0.42 | 8.91 ± 1.42 |
| b) Piretanid 20 mg/Ratte p.c (n = 6) | 1 - 5 Std. | 50.2 ± 8.3 | 6.92 ± 0.94 | 1.84 ± 0.42 | 6.83 ± 1.09 |
| | 6 - 24 Std. | 26.5 ± 3.9 | 3.46 ± 0.78 | 4.19 ± 0.78 | 2.82 ± 0.65 |
| | 1 - 24 Std. | 76.7 ± 7.4 | 10.38 ± 0.97 | 6.03 ± 1.03 | 9.68 ± 0.81 |

Tabelle 3

**Salidiuretische Wirkung von Piretanid nach oraler Gabe an der Ratte**

| Präparat | Sammel-perioden | Urin ml/kg | Natrium mmol/kg | Kalium mmol/kg | Chlorid mmol/kg |
|---|---|---|---|---|---|
| Piretanid 20 mg/Ratte p.o. (n = 2) | 1 - 5 Std. | 65.0 | 7.07 | 1.92 | 8.36 |
| | 6 - 24 Std. | 47.1 | 4.71 | 9.75 | 3.00 |
| | 1 - 24 Std. | 112.1 | 11.78 | 11.67 | 11.36 |

## Beispiel 2

### Zusammensetzung für eine Dosiereinheit

| | | |
|---|---|---|
| a) | Furosemid-Na | 5.3 mg |
| | Caprinsäurediethanolamid | 8.0 mg |
| | Caprinsäureethylester | 72.0 mg |
| | | 85.3 mg |

| | | |
|---|---|---|
| b) | Furosemid-Na | 21.3 mg |
| | Caprinsäurediethanolamid | 8.0 mg |
| | Caprinsäureethylester | 72.0 mg |
| | | 101.3 mg |

In Tabelle 4 ist die salidiuretische Wirkung nach percutaner Applikation von Furosemid-Natriumsalz in Dosen von 5.3 mg (Zusammensetzung a) und 21.3 mg (Zusammensetzung b) pro Ratte wiedergegeben. Als Vergleich diente die Ausscheidungsgröße nach oraler Gabe (Furosemid suspendiert in 2%iger wäßriger Stärkelösung) von 5 mg Furosemid pro Ratte (Tabelle 5).

Die TTS-Furosemid-Formulierungen zeigten nach percutaner Applikation eine dosisabhängige salidiuretische Wirkung. Darüberhinaus weit der Blutspiegel von Furosemid deutliche dosisabhängige Konzentrationsunterschiede auf (vgl. Tabelle 6), wodurch die dosisabhängige salidiuretische Wirkung zu erklären ist.

Tabelle 4

**Salidiuretische Wirkung von TTS - Furosemid - Natriumsalz an der Ratte**

| Präparat | Sammel-perioden | Urin ml/kg | Natrium mmol/kg | Kalium mmol/kg | Chlorid mmol/kg |
|---|---|---|---|---|---|
| a) Furosemid-Na 5.3mg/Ratte p.c. (n = 2) | 1 - 5 Std. | 81.0 | 8.24 | 2.52 | 10.10 |
| | 6 - 24 Std. | 27.5 | 2.39 | 5.29 | 1.01 |
| | 1 - 24 Std. | 108.5 | 10.63 | 7.81 | 11.11 |
| b) Furosemid-Na 21.3 mg/Ratte p.c. (n = 2) | 1 - 5 Std. | 94.2 | 9.75 | 2.89 | 11.76 |
| | 6 - 24 Std. | 45.2 | 3.00 | 7.82 | 2.35 |
| | 1 - 24 Std. | 139.4 | 12.75 | 10.71 | 13.11 |

Tabelle 5

Salidiuretische Wirkung von Furosemid nach oraler Gabe an der Ratte

| Präparat | Sammel-perioden | Urin ml/kg | Natrium mmol/kg | Kalium mmol/kg | Chlorid mmol/kg |
|---|---|---|---|---|---|
| Furosemid 5 mg/Ratte p.o. (n = 4) | 1 - 5 Std. | 47.8 ± 3.5 | 4.81 ± 0.75 | 1.40 ± 0.16 | 6.10 ± 0.62 |
| | 6 - 24 Std. | 11.9 ± 3.6 | 1.22 ± 0.21 | 2.48 ± 0.79 | 0.49 ± 0.11 |
| | 1 - 24 Std. | 59.7 ± 6.2 | 6.03 ± 0.75 | 3.88 ± 0.88 | 6.59 ± 0.61 |

Tabelle 6

Blutspiegel (µg/ml) von Furosemid-Natrium nach p.c. Applikation an der behaarten Ratte (n = 6)

| Std. | Zusammensetzung b) 21.3 mg/Ratte | Zusammensetzung a) 5.3 mg/Ratte |
|---|---|---|
| 0.5 | 0.25 ± 0.23 | 0.25 ± 0.14 |
| 1 | 2.18 ± 3.45 | 1.20 ± 1.31 |
| 2 | 27.13 ± 28.02 | 6.62 ± 4.78 |
| 4 | 89.70 ± 27.36 | 9.17 ± 5.56 |
| 6 | 52.05 ± 19.41 | 4.68 ± 2.60 |
| 24 | 0.95 ± 0.60 | 0.46 ± 0.37 |
| 48 | 0.04 ± 0.03 | 0 |
| Kontrolle | 0 | 0 |

## Beispiel 3

| | |
|---|---|
| Furosemid | 20,0 mg |
| Kokosfettsäurediethanolamid (= Gemisch aus Laurinsäurediethanolamid, Myristinsäurediethanolamid und Palmitinsäurediethanolamid) | 16,0 mg |
| Eicosen/Docosensäure-Eicosanol/Docosanol-ester (Jojobaöl) | 64,0 mg |
| | 100,0 mg |

## Beispiel 4

| | |
|---|---|
| Furosemid-Na | 5,3 mg |
| Caprinsäure-mono-iso-butandiolamid | 4,0 mg |
| Caprinsäureethylester | 72,0 mg |
| | 81,3 mg |

## Beispiel 5

| | |
|---|---|
| Furosemid-Na | 5,3 mg |
| Caprinsäure-N-ethyl-N-2-hydroxyethyl-amid | 4,0 mg |
| Caprinsäureethylester | 72,0 mg |
| | 81,3 mg |

Beispiel 6

| | |
|---|---|
| Furosemid-Na | 5,3 mg |
| Caprinsäurediethanolamid | 8,0 mg |
| Caprinsäureethylester | 14,4 mg |
| Essigsäuredecylester | 57,6 mg |
| | 85,3 mg |

Beispiel 7

| | |
|---|---|
| Furosemid-Na | 5,3 mg |
| Undecylensäure-di-ethanolamid | 4,0 mg |
| Caprinsäureethylester | 72,0 mg |
| | 81,3 mg |

Beispiel 8

| | |
|---|---|
| Furosemid-Na | 5,3 mg |
| Caprinsäure-di-iso-propanolamid | 4,0 mg |
| Caprinsäureethylester | 72,0 mg |
| | 81,3 mg |

Beispiel 9

| | |
|---|---|
| Furosemid-Na | 5,3 mg |
| Caprinsäure-mono-n-propanolamid | 4,0 mg |
| Caprinsäureethylester | 72,0 mg |
| | 81,3 mg |

Beispiel 10

| | |
|---|---|
| Furosemid-Na | 5,3 mg |
| Caprinsäure-2-ethyl-1,3-propandiol-2-amid | 4,0 mg |
| Caprinsäureethylester | 72,0 mg |
| | 81,3 mg |

Beispiel 11

| | |
|---|---|
| Furosemid-Na | 5,3 mg |
| Iso-nonanoylamino-trishydroxymethyl-methan | 4,0 mg |
| Caprinsäureethylester | 72,0 mg |
| | 81,3 mg |

Beispiel 12

| | |
|---|---|
| Furosemid-Na | 5,3 mg |
| Caprinsäurediethanolamid | 4,0 mg |
| Capryl/Caprinsäure-1,2-propandiol-diester | 72,0 mg |
| | 81,3 mg |

Beispiel 13

| | |
|---|---|
| Furosemid-Na | 5,3 mg |
| Caprinsäurediethanolamid | 4,0 mg |
| Essigsäure-1,12-dodecandiol-diester | 72,0 mg |
| | 81,3 mg |

Beispiel 14

| | |
|---|---|
| Furosemid-Na | 5,3 mg |
| Caprinsäurediethanolamid | 4,0 mg |
| Essigsäure-1,2-hexadecandiol-diester | 72,0 mg |
| | 81,3 mg |

Beispiel 15

| | |
|---|---|
| Furosemid-Na | 5,3 mg |
| Caprinsäurediethanolamid | 4,0 mg |
| Caprinsäure-diethylenglycol-diester | 72,0 mg |
| | 81,3 mg |

Beispiel 16

| | |
|---|---|
| Furosemid-Na | 5,3 mg |
| Caprinsäurediethanolamid | 4,0 mg |
| Laurinsäure-triethylenglycol-diester | 72,0 mg |
| | 81,3 mg |

Beispiel 17

| | |
|---|---|
| Furosemid-Na | 5,3 mg |
| Caprinsäurediethanolamid | 4,0 mg |
| Propionsäure-dipropylenglycolmonoester-monomyristyläther | 72,0 mg |
| | 81,3 mg |

## Beispiel 18

| | |
|---|---|
| Furosemid | 20,0 mg |
| Ölsäurediethanolamid | 16,0 mg |
| Ölsäureethylester | 64,0 mg |
| | 100,0 mg |

In der folgenden Tabelle 7 ist die salidiuretische Wirkung nach percutaner Applikation von Furosemid bzw. Furosemid-Natriumsalz aus Zusammensetzungen gemäß Beispiel 3 bis 18 pro Ratte wiedergegeben:

Tabelle 7

Salidiuretische Wirkung von TTS - Furosemid bzw. Furosemid - Natriumsalz an der Ratte

| Präparat | Sammel-perioden | Urin ml/kg | Natrium mmol/kg | Kalium mmol/kg | Chlorid mmol/kg |
|---|---|---|---|---|---|
| Beispiel 3 | 1 - 5 Std. | 15·2 | 0·66 | 0·96 | 0·76 |
| Furosemid | 6 - 24 Std. | 78·6 | 11·06 | 5·49 | 10·25 |
| 20,0 mg/Ratte p.c. | 1 - 24 Std. | 93·8 | 11·72 | 6·45 | 11·01 |
| (n=6) | | | | | |
| Beispiel 4 | 1 - 5 Std. | 36·9 | 3·48 | 1·57 | 4·10 |
| Furosemid-Na | 6 - 24 Std. | 50·9 | 5·90 | 5·39 | 4·89 |
| 5,3 mg/Ratte p.c. | 1 - 24 Std. | 87·8 | 9·38 | 6·96 | 8·99 |
| (n=6) | | | | | |
| Beispiel 5 | 1 - 5 Std. | 22·8 | 1·87 | 0·90 | 2·07 |
| Furosemid-Na | 6 - 24 Std. | 47·5 | 6·08 | 3·82 | 5·29 |
| 5,3 mg/Ratte p.c. | 1 - 24 Std. | 70·3 | 7·95 | 4·72 | 7·36 |
| (n=6) | | | | | |
| Beispiel 6 | 1 - 5 Std. | 66·5 | 7·21 | 1·99 | 8·55 |
| Furosemid-Na | 6 - 24 Std. | 25·1 | 2·29 | 5·31 | 0·93 |
| 5,3 mg/Ratte p.c. | 1 - 24 Std. | 91·6 | 9·50 | 7·30 | 9·48 |
| (n=6) | | | | | |
| Beispiel 7 | 1 - 5 Std. | 45·9 | 4·04 | 1·96 | 4·97 |
| Furosemid-Na | 6 - 24 Std. | 32·7 | 4·63 | 4·66 | 2·89 |
| 5,3 mg/Ratte p.c. | 1 - 24 Std. | 78·6 | 8·67 | 6·62 | 7·86 |
| (n=6) | | | | | |

Tabelle 7 (Fortsetzung)

| Präparat | Sammel-perioden | Urin ml/kg | Natrium mmol/kg | Kalium mmol/kg | Chlorid mmol/kg |
|---|---|---|---|---|---|
| Beispiel 8 | 1 - 5 Std. | 26·2 | 2·20 | 1·33 | 2·68 |
| Furosemid-Na | 6 - 24 Std. | 35·4 | 4·42 | 5·90 | 4·42 |
| 5,3 mg/Ratte p.c. | 1 - 24 Std. | 61·6 | 6·62 | 7·23 | 7·10 |
| (n=6) | | | | | |
| Beispiel 9 | 1 - 5 Std. | 25·8 | 1·84 | 1·38 | 2·12 |
| Furosemid-Na | 6 - 24 Std. | 29·9 | 3·88 | 4·72 | 2·98 |
| 5,3 mg/Ratte p.c. | 1 - 24 Std. | 55·7 | 5·72 | 6·10 | 5·10 |
| (n=6) | | | | | |
| Beispiel 10 | 1 - 5 Std. | 42·0 | 4·87 | 1·84 | 5·65 |
| Furosemid-Na | 6 - 24 Std. | 30·5 | 4·04 | 4·05 | 2·75 |
| 5,3 mg/Ratte p.c. | 1 - 24 Std. | 72·5 | 8·91 | 5·89 | 8·40 |
| (n=6) | | | | | |
| Beispiel 11 | 1 - 5 Std. | 52·8 | 5·59 | 1·64 | 6·19 |
| Furosemid-Na | 6 - 24 Std. | 25·8 | 3·68 | 4·20 | 2·39 |
| 5,3 mg/Ratte p.c. | 1 - 24 Std. | 78·6 | 9·27 | 5·84 | 8·58 |
| (n=6) | | | | | |
| Beispiel 12 | 1 - 5 Std. | 31·4 | 2·45 | 1·54 | 3·05 |
| Furosemid-Na | 6 - 24 Std. | 58·0 | 7·79 | 4·78 | 7·47 |
| 5,3 mg/Ratte p.c. | 1 - 24 Std. | 89·4 | 10·24 | 6·32 | 10·52 |
| (n=6) | | | | | |

Tabelle 7 (Fortsetzung)

| Präparat | Sammel-perioden | Urin ml/kg | Natrium mmol/kg | Kalium mmol/kg | Chlorid mmol/kg |
|---|---|---|---|---|---|
| Beispiel 13 | 1 - 5 Std. | 15·0 | 1·02 | 1·17 | 0·63 |
| Furosemid-Na | 6 - 24 Std. | 36·5 | 4·61 | 4·74 | 2·52 |
| 5,3 mg/Ratte p.c. (n=6) | 1 - 24 Std. | 51·5 | 5·63 | 5·91 | 3·15 |
| Beispiel 14 | 1 - 5 Std. | 19·9 | 1·27 | 1·43 | 1·41 |
| Furosemid-Na | 6 - 24 Std. | 57·9 | 7·88 | 4·83 | 6·81 |
| 5,3 mg/Ratte p.c. (n=6) | 1 - 24 Std. | 77·8 | 9·15 | 6·26 | 8·22 |
| Beispiel 15 | 1 - 5 Std. | 13·0 | 0·72 | 0·85 | 0·70 |
| Furosemid-Na | 6 - 24 Std. | 62·0 | 8·44 | 4·04 | 8·10 |
| 5,3 mg/Ratte p.c. (n=6) | 1 - 24 Std. | 75·0 | 9·16 | 4·89 | 8·80 |
| Beispiel 16 | 1 - 5 Std. | 35·2 | 1·96 | 1·52 | 3·55 |
| Furosemid-Na | 6 - 24 Std. | 50·0 | 6·90 | 5·01 | 6·00 |
| 5,3 mg/Ratte p.c. (n=6) | 1 - 24 Std. | 85·2 | 8·86 | 6·53 | 9·55 |
| Beispiel 17 | 1 - 5 Std. | 58·9 | 6·50 | 2·40 | 7·23 |
| Furosemid-Na | 6 - 24 Std. | 38·9 | 4·91 | 5·81 | 3·04 |
| 5,3 mg/Ratte p.c. (n=6) | 1 - 24 Std. | 97·8 | 11·41 | 8·21 | 10·27 |
| Beispiel 18 | 1 - 5 Std. | 17·6 | 0·84 | 0·95 | 1·11 |
| Furosemid | 6 - 24 Std. | 53·6 | 8·05 | 5·46 | 7·60 |
| 20,0 mg/Ratte p.c. (n=6) | 1 - 24 Std. | 71·2 | 8·89 | 6·41 | 8·71 |

## Beispiel 19

| | |
|---|---|
| Furosemid-Na | 21,3 mg |
| Caprinsäurediethanolamid | 4,0 mg |
| Caprinsäureethylester | 70,0 mg |
| Ölsäure-propantriol-mono-diester : | 1,0 mg |
| Wasser | 1,0 mg |
| | 97,3 mg |

In der folgenden Tabelle 8 sind Blutspiegel, Harnspiegel und salidiuretische Wirkung für die Zusammensetzung gemäß Beispiel 19 zusammengefaßt :

Tabelle 8

**Blutspiegel, Urinspiegel und salidiuretische Wirkung von Furosemid-Natrium nach p.c. Applikation an der behaarten Ratte (n=6)**

| Präparat | Blut | | Urin | | | | | |
|---|---|---|---|---|---|---|---|---|
| | Zeit [h] | Blutspiegel [µg/ml] | Sammelperiode [h] | Diurese [ml/kg] | Urinspiegel [µg/Sammelp.] | Na [mmol/kg] | K [mmol/kg] | Cl [mmol/kg] |
| | 1 | 0,64 | | | | | | |
| | 2 | 20,84 | 1 - 5 | 72,9 | 6180 | 7,7 | 2,9 | 9,1 |
| | 4 | 84,59 | | | | | | |
| Beispiel 19 | 6 | 57,24 | | | | | | |
| Furosemid- | 24 | 0,47 | 6 - 24 | 42,2 | 15080 | 3,8 | 6,9 | 2,6 |
| Na | 30 | 0,21 | | | | | | |
| 21,3 mg/ | 48 | 0 | 25 - 48 | 112,4 | 333 | 0,3 | 7,7 | 0,2 |
| Ratte p.c. | 49 | 0 | | | | | | |
| | 72 | 0 | 49 - 72 | 45,5 | 15 | 0,2 | 10,1 | 0,4 |

## Ansprüche

### Patentansprüche für die Vertragsstaaten : BE, CH, DE, FR, GB, IT, LI, LU, NL, SE

1. Zusammensetzung für transdermale therapeutische Systeme, dadurch gekennzeichnet, daß sie als Wirkstoff Furosemid, Piretanid oder Lemidosul oder ein physiologisch verträgliches Salz dieser Schleifendiuretika in einer Grundlage bestehend aus einem Fettsäurealkanolamid oder einem Fettsäurealkanolamidgemisch mit 8-18 Kohlenstoffatomen im Fettsäurerest und 2 bis 6 Kohlenstoffatomen und 1 bis 3 Hydroxygruppen im Alkanolamidrest und gegebenenfalls einem Carbonsäureester oder einem Carbonsäureestergemisch mit jeweils 1-22. Kohlenstoffatomen im Carbonsäureteil und 1-22 Kohlenstoffatomen im Alkoholteil enthält.

2. Zusammensetzung gemäß Anspruch 1, dadurch gekennzeichnet, daß die Grundlage aus einem Fettsäuredialkanolamid mit 8-12 Kohlenstoffatomen im Fettsäurerest und 2 bis 3 Kohlenstoffatomen im Alkanolrest und gegebenenfalls einem Fettsäureester oder einem Fettsäureestergemisch mit jeweils insgesamt 6 bis 30 Kohlenstoffatomen besteht.

3. Zusammensetzung gemäß Anspruch 1, dadurch gekennzeichnet, daß der Esteranteil maximal 98 Gew.-% bezogen auf das Gesamtgewicht der Zusammensetzung beträgt.

4. Zusammensetzung gemäß Anspruch 1, dadurch gekennzeichnet, daß die Grundlage aus einem Fettsäurealkanolamid und einem Carbonsäurester besteht.

5. Zusammensetzung gemäß Anspruch 1, dadurch gekennzeichnet, daß die Grundlage aus einem Fettsäurediethanolamid und einem Carbonsäureester oder Carbonsäureestergemisch besteht.

6. Zusammensetzung gemäß Anspruch 1, dadurch gekennzeichnet, daß die Grundlage aus Caprinsäurediethanolamid und Caprinsäureethylester besteht.

7. Zusammensetzung gemäß Anspruch 1, dadurch gekennzeichnet, daß die Grundlage aus Laurinsäurediethanolamid und Laurinsäurehexylester besteht.

8. Zusammensetzung gemäß Anspruch 1, dadurch gekennzeichnet, daß sie 1 bis 50 Gew.-% Wirkstoff, bezogen auf das Gesamtgewicht der Zusammensetzung, enthält.

9. Zusammensetzung gemäß Anspruch 1, dadurch gekennzeichnet, daß sie noch inerte Füllstoffe und übliche Hilfsstoffe enthält.

10. Verfahren zur Herstellung einer Zusammensetzung gemäß Anspruch 1, dadurch gekennzeichnet, daß man Furosemid, Piretanid oder Lemidosul oder ein physiologisch verträgliches Salz dieser Schleifendiuretika mit einem Fettsäurealkanolamid oder einem Fettsäurealkanolamidgemisch mit 8 bis 18 Kohlenstoffatomen im Fettsäurerest und 2 bis 6 Kohlenstoffatomen und 1 bis 3 Hydroxygruppen im Alkanolamidrest und gegebenenfalls einem Carbonsäureester oder einem Carbonsäureestergemisch mit jeweils 1-22 Kohlenstoffatomen im Carbonsäureteil und 1-22 Kohlenstoffatomen im Alkoholteil bei Temperaturen zwischen Raumtemperatur und 50°C innig vermischt.

11. Verwendung einer Zusammensetzung gemäß Anspruch 1 in einem transdermalen therapeutischem System.

### Patentansprüche für die Vertragsstaaten : AT, GR, ES

1. Verfahren zur Herstellung einer Zusammensetzung für transdermale therapeutische Systeme, dadurch gekennzeichnet, daß man Furosemid, Piretanid oder Lemidosul oder ein physiologisch verträgliches Salz dieser Schleifendiuretika mit einem Fettsäurealkanolamid oder einem Fettsäurealkanolamidgemisch mit 8 bis 18 Kohlenstoffatomen im Fettsäurerest und 2 bis 6 Kohlenstoffatomen und 1 bis 3 Hydroxygruppen im Alkanolamidrest und gegebenenfalls einem Carbonsäureester oder einem Carbonsäureestergemisch mit jeweils 1-22 Kohlenstoffatomen im Carbonsäureteil und 1-22 Kohlenstoffatomen im Alkoholteil bei Temperaturen zwischen Raumtemperatur und 50°C innig vermischt.

2. Verfahren zur Herstellung einer Zusammensetzung gemäß Anspruch 1, dadurch gekennzeichnet, daß man eine Grundlage aus einem Fettsäuredialkanolamid mit 8-12 Kohlenstoffatomen im Fettsäurerest und 2 bis 3 Kohlenstoffatomen im Alkanolrest und gegebenenfalls einem Fettsäureester oder einem Fettsäureestergemisch mit jeweils insgesamt 6 bis 30 Kohlenstoffatomen verwendet.

3. Verfahren zur Herstellung einer Zusammensetzung gemäß Anspruch 1, dadurch gekennzeichnet, daß in der Grundlage der Esteranteil maximal 98 Gew.-% bezogen auf das Gesamtgewicht der Zusammensetzung beträgt.

4. Verfahren zur Herstellung einer Zusammensetzung gemäß Anspruch 1, dadurch gekennzeichnet, daß man eine Grundlage bestehend aus einem Fettsäurealkanolamid und einem Carbonsäurester verwendet.

5. Verfahren zur Herstellung einer Zusammensetzung gemäß Anspruch 1, dadurch gekennzeichnet, daß man eine Grundlage bestehend aus einem Fettsäurediethanolamid und einem Carbonsäureester oder Carbonsäureestergemisch verwendet.

6. Verfahren zur Herstellung einer Zusammensetzung gemäß Anspruch 1, dadurch gekennzeichnet, daß man eine Grundlage bestehend aus Caprinsäurediethanolamid und Caprinsäureethylester verwendet.

7. Verfahren zur Herstellung einer Zusammensetzung gemäß Anspruch 1, dadurch gekennzeichnet, daß man eine Grundlage bestehend aus Laurinsäurediethanolamid und Laurinsäurehexylester verwendet.

8. Verfahren zur Herstellung einer Zusammensetzung gemäß Anspruch 1, dadurch gekennzeichnet, daß sie 1 bis 50 Gew.-% Wirkstoff bezogen auf das Gesamtgewicht der Zusammensetzung enthält.

9. Verfahren zur Herstellung einer Zusammensetzung gemäß Anspruch 1, dadurch gekennzeichnet, daß man noch inerte Füllstoffe und übliche Hilfsstoffe zusetzt.

## Claims

### Claims for the Contracting States : BE, CH, DE, FR, GB, IT, LI, LU,, NL, SE

1. A composition for transdermal therapeutic systems, which contains as active compound furosemide, piretanide or lemidosul, or a physiologically tolerated salt of these loop diuretics, in a base composed of a fatty acid alkanolamide, or of a fatty acid alkanolamide mixture, in each case having 8-18 carbon atoms in the fatty acid residue and 2 to 6 carbon atoms and 1 to 3 hydroxyl groups in the alkanolamide residue, and, where appropriate, of a carboxylic ester, or of a carboxylic ester mixture, in each case having 1-22 carbon atoms in the carboxylic acid moiety and 1-22 carbon atoms in the alcohol moiety.

2. A composition as claimed in claim 1, wherein the base is composed of a fatty acid dialkanolamide having 8-12 carbon atoms in the fatty acid residue and 2 to 3 carbon atoms in the alkanol residue, and, where appropriate, of a fatty acid ester, or of a fatty acid ester mixture, in each case having a total of 6 to 30 carbon atoms.

3. A composition as claimed in claim 1, wherein the ester proportion amounts to a maximum of 98% by weight based on the total weight of the composition.

4. A composition as claimed in claim 1, wherein thebase is composed of a fatty acid alkanolamide and of a carboxylic ester.

5. A composition as claimed in claim 1, wherein the base is composed of a fatty acid diethanolamide and of a carboxylic ester or carboxylic ester mixture.

6. A composition as claimed in claim 1, wherein the base is composed of capric acid diethanolamide and ethyl caprate.

7. A composition as claimed in claim 1, wherein the base is composed of lauric acid diethanolamide and hexyl laurate.

8. A composition as claimed in claim 1, which contains 1 to 50% by weight of active compound based on the total weight of the composition.

9. A composition as claimed in claim 1, which also contains inert vehicles and customary auxiliaries.

10. A process for the preparation of a composition as claimed in claim 1, which comprises intimately mixing furosemide, piretanide or lemidosul, or a physiologically tolerated salt of these loop diuretics, with a fatty acid alkanolamide, or with a fatty acid alkanolamide mixture, in each case having 8 to 18 carbon atoms in the fatty acid residue and 2 to 6 carbon atoms and 1 to 3 hydroxyl groups in the alkanolamide residue and, where appropriate, with a carboxylic ester, or with a carboxylic ester mixture, in each case having 1-22 carbon atoms in the carboxylic acid moiety and 1-22 carbon atoms in the alcohol moiety, at temperatures between room temperature and 50°C.

11. The use of a composition as claimed in claim 1 in a transdermal therapeutic system.

### Claims for the Contracting States : AT, GR, ES

1. A process for the preparation of a composition for transdermal therapeutic systems, which comprises intimately mixing furosemide, piretanide or lemidosul, or a physiologically tolerated salt of these loop diuretics, with a fatty acid alkanolamide, or with a fatty acid alkanolamide mixture, in each case having 8 to 18 carbon atoms in the fatty acid residue and 2 to 6 carbon atoms and 1 to 3 hydroxyl groups in the alkanolamide residue and, where appropriate, with a carboxylic ester, or with a carboxylic ester mixture, in each case having 1-22 carbon atoms in the carboxylic acid moiety and 1-22 carbon atoms in the alcohol moiety, at temperatures between room temperature and 50°C.

2. The process for the preparation of a composition as claimed in claim 1, wherein a base composed of a fatty acid dialkanolamide having 8-12 carbon atoms in the fatty acid residue and 2 to 3 carbon atoms in the alkanol residue and, where appropriate,, of a fatty acid ester, or of a fatty acid ester mixture, in each case having a total of 6 to 30 carbon atoms, is used.

3. The process for the preparation of a composition as claimed in claim 1, wherein the ester proportion in the base amounts to a maximum of'98% by weight based on the total weight of the composition.

4. The process for the preparation of a composition as claimed in claim 1, wherein a base composed of a fatty acid alkanolamide and of a carboxylic ester is used.

5. The process for the preparation of a composition as claimed in claim 1, wherein a base composed of a fatty acid diethanolamide and of a carboxylic ester or carboxylic ester mixture is used.

6. The process for the preparation of a composition as claimed in claim 1, wherin a base composed of capric acid diethanolamide and ethyl caprate is used.

7. The process for the preparation of a composition as claimed in claim 1, wherein a base composed of lauric acid diethanolamide and hexyl laurate is used.

8. The process for the preparation of a composition as claimed in claim 1, which contains 1 to 50% by weight of active compound based on the total weight of the composition.

9. The process for the preparation of a composition as claimed in claim 1, wherein inert vehicles and customary auxiliaries are also added.

**Revendications**

**Revendications pour les Etats Contractants : BE, CH, DE, FR, GB, IT, LI, LU, NL, SE**

1. Composition pour des systèmes thérapeutiques transdermiques, caractérisée en ce qu'elle renferme, comme principe actif, du furosémide, du pirétanide ou du lémidosul ou un sel physiologiquement acceptable de ces diurétiques de l'anse, dans une base comprenant un alcanolamide d'acide gras ou un mélange d'alcanolamides d'acides gras comportant 8 à 18 atomes de carbone dans le reste acide gras et 2 à 6 atomes de carbone et 1 à 3 groupes hydroxy dans le reste alcanolamide, et éventuellement un ester d'acide carboxylique ou un mélange d'esters d'acides carboxyliques comportant chacun 1 à 22 atomes de carbone dans la partie acide carboxylique et 1 à 22 atomes de carbone dans la partie alcool.

2. Composition selon la revendication 1, caractérisée en ce que la base comprend un dialcanolamide d'acide gras comportant 8 à 12 atomes de carbone dans le reste acide gras et 2 à 3 atomes de carbone dans le reste alcanol, et éventuellement un ester d'acide gras ou un mélange d'esters d'acides gras comportant chacun au total 6 à 30 atomes de carbone.

3. Composition selon la revendication 1, caractérisée en ce que la partie ester représente au maximum 98% en poids, par rapport au poids total de la composition.

4. Composition selon la revendication 1, caractérisée en ce que la base comprend un alcanolamide d'acide gras et un ester d'acide carboxylique.

5. Composition selon la revendication 1, caractérisée en ce que la base comprend un diéthanolamide d'acide gras et un ester d'acide carboxylique ou un mélange d'esters d'acides carboxyliques.

6. Composition selon la revendication 1, caractérisée en ce que la base comprend le diéthanolamide de l'acide caprique et l'ester éthylique de l'acide caprique.

7. Composition selon la revendication 1, caractérisée en ce que la base comprend le diéthanolamide de l'acide laurique et l'ester hexylique de l'acide laurique.

8. Composition selon la revendication 1, caracterisée en ce qu'elle renferme de 1 à 50% en poids de principe actif, par rapport au poids total de la composition.

9. Composition selon la revendication 1, caractérisée en ce qu'elle renferme encore des charges inertes et adjuvants usuels.

10. Procédé de fabrication d'une composition selon la revendication 1, caractérisé en ce que l'on mélange intimement, à des températures allant de la température ambiante à 50°C, du furosémide, du pirétanide ou du lémidosul ou un sol physiologiquement acceptable de ces diurétiques de l'anse, avec un alcanolamide d'acide gras ou un mélange d'alcanolamides d'acides gras comportant 8 à 18 atomes de carbone dans le reste acide gras et 2 à 6 atomes de carbone et 1 à 3 groupes hydroxy dans le reste alcanolamide, et éventuellement avec un ester d'acide carboxylique ou un mélange d'esters d'acides carboxyliques comportant chacun 1 à 22 atomes de carbone dans la partie acide carboxylique et 1 à 22 atomes de carbone dans la partie alcool.

11. Utilisation d'une composition selon la revendication 1 dans un système thérapeutique transdermi-

que.

**Revendications pour les Etats contractants : AT, GR, ES**

1. Procédé de fabrication d'une composition pour des systèmes thérapeutiques transdermiques caractérisé en ce que l'on mélange intimement, à des températures allant de la température ambiante à 50°C, du furosémide, du pirétanide ou du lémidosul ou un sel physiologiquement acceptable de ces diurétiques de l'anse, avec un alcanolamide d'acide gras ou un mélange d'alcanolamides d'acides gras comportant 8 à 18 atomes de carbone dans le reste acide gras et 2 à 6 atomes de carbone et 1 à 3 groupes hydroxy dans le reste alcanolamide, et éventuellement avec un ester d'acide carboxylique ou un mélange d'esters d'acides carboxyliques comportant chacun 1 à 22 atomes de carbone dans la partie acide carboxylique et 1 à 22 atomes de carbone dans la partie alcool.

2. Procédé de fabrication d'une composition selon la revendication 1, caractérisé en ce que l'on utilise une base comprenant un dialcanolamide d'acide gras comportant 8 à 12 atomes de carbone dans le reste acide gras et 2 à 3 atomes de carbone dans le reste alcanol, et éventuellement un ester d'acide gras ou un mélange d'esters d'acides gras comportant chacun au total 6 à 30 atomes de carbone.

3. Procédé de fabrication d'une composition selon la revendication 1, caractérisé en ce que, dans la base, la partie ester représente au maximum 98% en poids, par rapport au poids total de la composition.

4. Procédé de fabrication d'une composition selon la revendication 1, caractérisé en ce que l'on utilise une base comprenant un alcanolamide d'acide gras et un ester d'acide carboxylique.

5. Procédé de fabrication d'une composition selon la revendication 1, caractérisé en ce que l'on utilise une base comprenant un diéthanolamide d'acide gras et un ester d'acide carboxylique ou un mélange d'esters d'acides carboxyliques.

6. Procédé de fabrication d'une composition selon la revendication 1, caractérisé en ce que l'on utilise une base comprenant le diéthanolamide de l'acide caprique et l'ester éthylique de l'acide caprique.

7. Procédé de fabrication d'une composition selon la revendication 1, caractérisé en ce que l'on utilise une base comprenant le diéthanolamide de l'acide laurique et l'ester hexylique de l'acide laurique.

8. Procédé de fabrication d'une composition selon la revendication 1, caractérisé en ce qu'elle renferme de 1 à 50% en poids de principe actif, par rapport au poids total de la composition.

9. Procédé de fabrication d'une composition selon la revendication 1, caractérisé en ce qu'elle renferme encore des charges inertes et adjuvants usuels.